# EUROPÄISCHE PATENTSCHRIFT

(11) **EP 0 924 208 B1**
(45) Veröffentlichungstag und Bekanntmachung des Hinweises auf die Patenterteilung: **05.03.2003**
(21) Anmeldenummer: 98123135.0
(22) Anmeldetag: 04.12.1998
(51) Int. Cl.: C07D 311/72

(54) **Verfahren zur Herstellung von Alpha-Tocopherolestern**
Process for the preparation fo alpha-Tocopherol esters
Procédé pour la préparation des esters de l'alpha-tocophérol

(30) Priorität: 20.12.1997 DE 19757124
(43) Veröffentlichungstag der Anmeldung: 23.06.1999
(73) Patentinhaber: Degussa AG, 40474 Düsseldorf (DE)
(72) Erfinder: Krill, Steffen Dr., 67346 Speyer (DE); Hübner, Frank Dr., 64372 Ober-Ramstadt (DE); Hahn, Rainer Dr., 63654 Büdingen (DE); Weigel, Horst, 63517 Rodenbach (DE); Huthmacher, Klaus Dr., 63571 Gelnhausen (DE)

(56) Entgegenhaltungen:
- EP-A- 0 694 541
- FR-A- 2 259 822
- US-A- 3 789 086

## Beschreibung

Die Erfindung betrifft die Herstellung von α-DL-Tocopherolestern (Vitamin E-Ester), seinen Derivaten und Homologen durch Umsetzung von diacetylierten substituierten Hydrochinonen mit Phytol und Isophytol oder dessen Homologen in Gegenwart von katalytisch wirkenden Verbindungen.
Bekannt sind vor allem Verfahren zur Herstellung von α-DL-Tocopherol.
α-Tocopherol und seine Derivate sind als Futtermitteladditive, Antioxidatien, Mittel zur Anregung des Blutkreislaufs, Mittel zur Verringerung der Zellalterung und für verwandte Anwendungen von Bedeutung.

Gemäß dem Stand der Technik geht man im allgemeinen von Trimethylhydrochinon (TMHQ) aus, das unter Verwendung verschiedener Katalysatorsysteme mit Isophytol umgesetzt wird. (DE-OS 4243464 = US-PS 5,523,420, DE-OS 19603142, EP 0694 541).
Nach beendeter Reaktion muß das Produkt anschließend vollständig acetyliert werden, um z. B. zum lagerstabilen, handelsüblichen Vitamin-E-Acetat zu gelangen. All diesen Verfahren ist gemeinsam, daß sie nicht direkt zu Tocopherolacetat, der lagerstabilen, handelsüblichen Vitamin E Form gelangen.
Es hat auch Versuche gegeben, Trimethylhydrochinonester als Edukt der Synthese von Tocopherolen mit Isophytol umzusetzen.
Die FR-A 2 259822 (DE-OS 2 404621) betrifft den Einsatz von diacetyliertem Trimethylhydrochinon.
Die dort beschriebene Kondensation mit Isophytol in Gegenwart einer festen Säure liefert jedoch nur eine Ausbeute von ca. 41 % α-DL-Tocopherol und führe nicht zu den entsprechenden Estern.
Die Kondensation von Trimethylhydrochinonmonoacetat mit Isophytol wird in der DE-OS 2 160 103 ( = US-PS 3,789,086) beschrieben.
Man erhält unter Verwendung von Fe(II)Cl₂ und Salzsäure bei gleichzeitiger Abtrennung des Reaktionswassers nur geringe Mengen des α-Tocopherolacetats und muß unter Aminkatalyse und Zugabe überstöchiometrischer Mengen an Acetanhydrid nachacetylieren.

Gemäß der JP-OS 51-80859 (15. Juli 1976) setzt man Trimethylhydrochinon oder dessen Ester mit Isophytol in Gegenwart von Zinkchlorid um.
Die Reaktion führt bei Temperaturen von mehr als 100 ° C entsprechend der Aufgabenstellung zum α-Tocopherol.

Aufgabe der Erfindung ist es, ein verbessertes Verfahren zur Herstellung von α-DL-Tocopherolestern zur Verfügung zu stellen, bei dem die Reaktion schon im ersten Schritt möglichst weit in Richtung des Esters abläuft.

Gegenstand der Erfindung ist ein Verfahren zur Herstellung von α-Tocopherolestern, seinen Derivaten oder -homologen gemäß der allgemeinen Formel 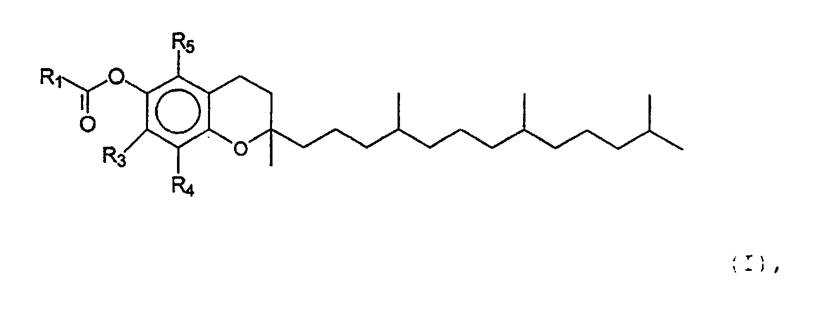 das dadurch gekennzeichnet ist, daß man den Mono- oder Diester eines Hydrochinons gemäß der allgemeinen Formel 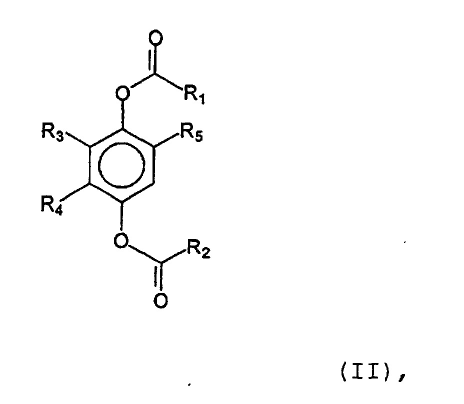 in der bedeuten:
- R₁, R₂:: Alkyl mit C₁-C₂₀, verzweigt oder unverzweigt,mit R₁ ungleich R₂ oder R₁ = R₂
- R₃, R₄, R₅:: H, Alkyl mit C₁-C₃, gleich oder verschieden,
mit einem Allylalkoholderivat der allgemeinen Formel 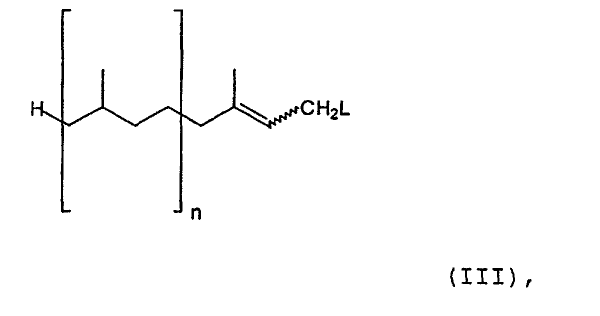 in der n eine Zahl von 0 bis 5 und L einer Hydroxyl-, Halogen-, Acetoxy-, Methansulfonyloxy-, Ethansulfonyloxy-, Benzolsulfonyloxy- oder Toluol-sulfonylgruppe entspricht, oder mit einem Allylalkohol der allgemeinen Formel 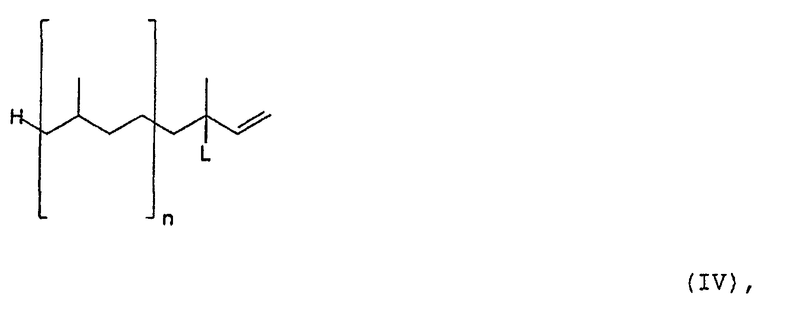 in der n die selben Zahlen repräsentiert wie oben und B einer Hydroxy-, Halogen- oder Acetoxygruppe entspricht, in Gegenwart von Zinkhalogeniden und Protonen abspaltenden Säuren und Wasser bei einer Temperatur von 25 bis 100 ° C, bevozugt 40 bis 80 ° C, zur Reaktion bringt.

Trimethylhydrochinon-Diacetat und Isophytol stehen als bevorzugt eingesetzte Reaktanden.
Das Diacetat wird in einem besonders geeigneten Verfahren gemäß EP-A 0808815 hergestellt.
In einer bevorzugten Ausführungsform legt man den gelösten Ester gemäß Formel (II) gemeinsam mit den Zinkhalogeniden und der Säure vor und setzt dieser Lösung die Verbindung gemäß den Formeln (III) oder (IV) zu.
Das bei der Reaktion sich bildende Wasser bzw. die Essigsäure verbleiben im Reaktionsgemisch und werden nicht ausgeschleust.
Es ist für den Fachmann unerwartet, daß die gewünschten Ester in so hoher Konzentration trotz des sauren Milieus und der Gegenwart von Wasser als Produkt gefunden werden. Nicht veresterte Verbindungen werden im Anschluß an die Umsetzung,gegebenenfalls nach den bekannten Verfahren in die gewünschten Ester überführt, insbesondere durch Acetylierung mit Acetanhydrid.
Besonders bewährt hat sich die Verfahrensweise, Wasser in Konzentrationen von 10⁻² Mol % bis 100 Mol %, bezogen auf eingesetztes TMHQ-Diacetat in der Reaktionsmischung einzustellen. Dies kann über die Zugabe und Dosierung von wässriger bzw. konzentrierter Protonensäure erfolgen.
Zu guten Ausbeuten gelangt man bei der Verwendung von Salzsäure als Protonensäure.
Als Säuren sind aber auch geeignet, Schwefelsäure, Schwefelsäure/SO₃ - Mischungen mit verschiedenen SO₃ Konzentrationen, Trifluormethansulfonsäure und entsprechende Supersäuren mit einem H₀-Wert kleiner gleich -11,9, insbesondere auch Gemische von Borsäure und Oxalsäure, im allgemeinen im einem molaren Verhältnis von 1 : 1 bis 1 :5, besonders 1 : 2.
Sie werden im allgemeinen in einer Konzentration 10⁻² bis 100 Mol %, bezogen auf TMHQ-Diacetat eingesetzt. Zinkhalogenide, insbesondere Zinkchlorid oder Zinkbromid werden besonders in einer Konzentration von 10 bis 100 Mol %, bezogen auf Trimethylhydrochinon-Diacetat verwendet.
Wenn auch keine besonderen Einschränkungen im Hinblick auf die Menge des eingesetzten Lösungsmittels vorhanden sind, arbeitet man bevorzugt bei 0,1 bis 100 ml/g TMHQ-Diacetat, wobei 1 bis 10 ml/g besonders geeignet sind.
In einer bevorzugten Ausführungsform legt man das Ausgangsprodukt gemäß Formel (II), die Katalysatormischung aus Lewissäure und Brönstedtsäure und das organische Lösungsmittel vor und setzt anschließend die Verbindungen gemäß den Formeln (III) oder (IV) zu.
Im Anschluß an die Umsetzung erfolgt die Abtrennung des gewünschten Produkts, gegebenenfalls auch die Veresterung nach den bekannten Methoden.

Geeignete organische Lösungsmittel für die Umsetzung sind Carbonatester, wie z. B.
Dimethylcarbonat,
Diethylcarbonat,
Dipropylcarbonat,
Methylethylcarbonat,
Ethylencarbonat und
Propylencarbonat
oder Carbonsäureester, wie z. B.
n-Propylacetat,
i-Propylacetat,
n-Butylacetat,
i-Butylacetat,
t-Butylacetat,
n-Amylacetat,
i-Amylacetat [CH₃COOCH₂CH₂CH(CH₃)₂],
sec-Amylacetat [CH₃COOCH(CH₃)CH₂CH₂CH₃],
t-Amylacetat [CH₃COOC(CH₃)₂CH₂CH₃],
2,2-Dimethylpropylacetat [CH₃COOCH₂C(CH₃)₃].
2-Methylbutylacetat [CH₃COOCH₂CH(CH₃)CH₂CH₃],
Methylpropionat,
n-Butylpropionat,
Ethylbutyrat,
i-Propylbutyrat,
Methylisobutyrat,
Ethylisobutyrat,
i-Butylisobutyrat,
Methylvalerat,
Ethylvalerat,
Methylisovalerat,
Ethylisovalerat,

Besonders bevorzugt sind n-Propylacetat, i-Propylacetat, n-Butylacetat, i-Butylacetat, n-Butylpropionat, Ethylbutyrat, i-Propylbutyrat, Methylisobutyrat, Ethylisobutyrat, Methylvalerat,
oder nicht polare Lösungsmittel wie z. B.
Pentan, Hexan, Heptan, Octan,
Ligroin, Petrolether, Cyclohexan,
Benzol, Toluol und Xylol.
oder aliphatische Alkohole, wie z. B.
Methanol,
Ethanol,
n-Propanol,
i-Propanol,
n-Butanol,
i-Butanol,
t-Butanol,
n-Amylalkohol (1-Pentanol),
2-Pentanol (1-Methyl-1-Butanol)
3-Pentanol (1-Ethyl-1-Propanol)
i-Amylalkohol (3-Methyl-1-Butanol)
t-Amylalkohol (1,1-Dimethyl-1-Propanol)
2,2-Dimethyl-1-Propanol,
1,2-Dimethyl-1-Propanol,
2-Methyl-1-Butanol, und
3-Methyl-2-Butanol.

Besonders bevorzugt sind n-Propanol, i-Propanol, n-Butanol, i-Butanol, t-Butanol, n-Amylalkohol, 2-Pentanol, 3-Pentanol, i-Amylalkohol und t-Amylalkohol, sowie Gemischen aus den genannten Gruppen von Lösungsmitteln.In Gemischen wirkt eines der Lösungsmittel als Cosolvens.

Einen besonderen Einfluß auf die Selektivität findet man bei Verwendung von cyclischen Carbonaten wie unter anderem Ethylen- oder Propylencarbonat, offenkettigen Estern der Essigsäure wie Ethylacetat, Propylacetat, Butylacetat und Isobutylacetat

Es ist ebenso möglich, die Cyclokondensation in flüssigem oder überkritischem Kohlendioxid als Solvens durchzuführen. Auch der kontinuierlichen Ausführung unter Recyclierung des Katalysators in einem geeigneten Lösungsmittel steht nichts im Wege.

Die vorteilhaften Ergebnisse der erfindungsgemäß einfach durchzuführenden Synthese von DL-α-Tocopherol bzw. - Tocopherolacetat aus Trimethylhydrochinondi- oder - monoacetat und Isophytol bzw.den in Anspruch 1 genannten Verbindungen in Anwesenheit einer Protonensäure und Zinkhalogeniden werden besonders deshalb nicht erwartet, da nach dem Stand der Technik üblicherweise das bei der Reaktion entstehende Kondensationswasser ausgekreist wird. Beim erfindungsgemäßen Verfahren spielt im Gegensatz hierzu jedoch Wasser eine katalytische Rolle und ist für den selektiven Reaktionsverlauf notwendig.

Das erfindungsgemäße Verfahren vereinfacht die gebräuchliche Verfahrensweise zusätzlich erheblich, da es sich zeigt, daß im Gegensatz zum Stand der Technik ein verestertes TMHQ eingesetzt wird, dessen Estergruppe sich zu einem unerwartet großen Teil im Endprodukt gemäß Formel (I) wiederfindet. Damit kann Acetanhydrid bei der sich anschließenden Acetylierung gespart werden.

Andererseits kann man auch in der Weise verfahren, daß das Rohprodukt der Reaktion verseift wird und als Endprodukt DL-α-Tocopherol entsteht.

Damit ist es gelungen, eine Tocopherolacetatsynthese bei Verkürzung der Reaktionsstufenzahlen und Einsparung von Rohstoffen auf ein technisch durchführbares, wirtschaftliches Niveau zu heben.

### Ausführungsbeispiele:

### Ausführungsbeispiel 1

### Synthese von α-DL-Tocopherolacetat:

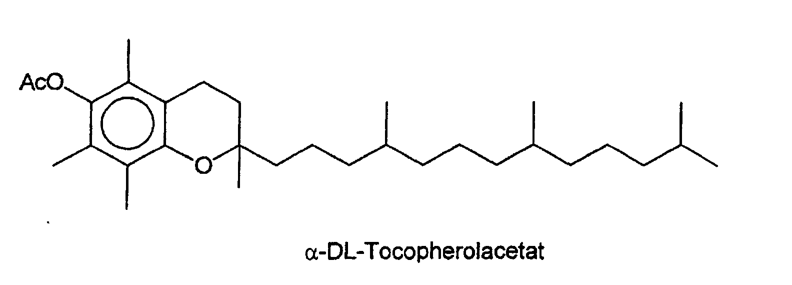

Eine Mischung von 10,9 g (= 46 mmol) Trimethylhydrochinon-1,4-Diacetat (GC: 100 %), 5,2 g (= 38,17 mmol = 83 Mol% bezogen auf TMHQ-DA) ZnCl₂ und 0,65 ml HCl_{konz} in 15 ml Hexan, 0,7 ml n-Butanol, und 15 ml n-Propylacetat wird auf 60 °C erhitzt. Zu dieser Lösung wird innerhalb von 4h 14,5 g(= 48 mmol) Isophytol (GC: > 98%) zugetropft. Nach beendeter Zugabe wird weitere 2h bei 60°C gerührt. Nach Beendigung der Reaktion kühlt man auf Raumtemperatur und gibt zu der erhaltenen Reaktionsmischung 100 ml Petrolether. Man wäscht die Lösung mit 2 x 20 ml Wasser und 2 x 20 ml gesättigter NaHCO₃-Lösung. Danach trennt man die organische Phase ab, trocknet über Magnesiumsulfat, und zieht nach Filtration des Salzes das Lösungsmittel am Rotationverdampfer ab. Man erhält 21 g eines gelben Öls, das laut HPLC Analyse die folgende Zusammensetzung hat: 28 wt% Vitamin E (=5,88g), 65,8 wt% Vitamin E-Acetat (= 13,82 g), 1,6 wt% (=0,34g) TMHQ-Monoacetat. Nach Verrechnung des Gehalts an freiem Vitamin E in Vitamin E-Acetat Equivalente ergibt sich eine Gesamtausbeute von 20,27 g Tocopherolacetat, was 93,2% der Theorie entspricht. Der Umsatz von TMHQ-Diacetat ist quantitativ.

### Ausführungsbeispiel 2

### Synthese von Tocopherolacetat aus TMHQ-DA

Man verfährt wie in Beispiel 1 beschrieben und legt 10,9 g (= 46 mmol) TMHQ-Diacetat in 15 ml Isobutylacetat vor. Zu dieser Lösung gibt man 0,63 ml konzentrierte Salzsäure und 5,2 g (= 38,17 mmol) ZnCl₂. Die Zugabe von Isophytol (14,5 g = 48 mmol) erfolgt bei 60 °C innerhalb von 6h. Nach üblicher Aufarbeitung gelangt man zu 21,7 g eines braunen Rohprodukts, das laut HPLC die folgende Zusammensetzung besitzt: 37,6 wt% Tocopherol und 54,4 wt% Tocopherolacetat. Der Umsatz von TMHQ-Diacetat beträgt 100%, an TMHQ-MA werden noch 0,74 wt% nachgewiesen. Dies entspricht einer Ausbeute von 11,8 g Vitamin E-Acetat und 8,16 g Vitamin E. Nach Umrechnung der Vitamin E Ausbeute in Vitamin E-Acetat äquivalente entspricht dies einer Ausbeute von 95,45%. Die Selektivität zu Vitamin E beträgt bei einem TMHQ-MA Umsatz von 98,17% 42%. Die Selektivität zu Vitamin E-Acetat beträgt 55,3%. Damit ergibt sich eine Gesamtselektivität zu den gewünschten Produkten von 97,3%.

### Ausführungsbeispiel 3

### Synthese von Tocopherolacetat aus TMHQ-MA:

Es wird gearbeitet wie im vorigen Beispiel 3, nur wird anstelle des Diacetats das aus diesem durch selektive Verseifung gewonnene TMHQ-Monoacetat eingesetzt. Man legt 5,2 g (38,17 mmol) ZnCl₂ und 8,9 g (= 45,8 mmol) TMHQ-Monoacetat in 15 ml Ethylacetat vor, fügt zu dieser Mischung 0,63 ml konzentrierte HCl hinzu und erhitzt auf 60 °C. Während 2,5 h werden kontinuierlich 13,8 g (= 46,5 mmol) Isophytol zugetropft. Man lässt weitere 2,5 h bei 50 °C nachreagieren. Nach üblicher Aufarbeitung erhält man ein braunes Öl, das laut quantitativer HPLC-Analyse folgende Zusammensetzung besitzt: 4,83 wt% TMHQ-MA, 18 wt% Vitamin E und 66,9 wt% Vitamin E-Acetat. Die Auswaage beträgt 21,0 g. Dies entspricht einer Ausbeute von 84%. Der Umsatz beträgt 88,6% und die Selektivität zu Tocopherol und Tocopherol-Acetat beträgt 94,8%.

### Ausführungsbeispiel 4

### Synthese von Tocopherolacetat aus TMHQ-DA:

Man arbeitet bezüglich der Zugabereihenfolge der Komponenten wie in Beispiel 1 und legt 5,2 g (38,17 mmol) ZnCl₂ und 0,65 ml konzentrierte HCl und 10,9 g (= 46,1 mmol) TMHQ-DA in 15 ml n-Propylacetat vor. Innerhalb von 4,5 h gibt man 14,5 g (= 48,9 mmol) Isophytol bei 50 °C zu und rührt 1,5 h nach. Nach üblicher Aufarbeitung erhält man 19,3 g eines honigfarbenen Öls, das nach HPLC Analytik die folgenden Gehalte an Tocopherol bzw. seiner acylierten Form hat: 15,8 wt% Vitamin E, 64,5 wt% Vitamin E-Acetat. Das entspricht bei Umrechnung des gebildeten Vitamin E auf die equivalente Menge Vitamin E-Acetat einer Ausbeute von 72,5%.

### Ausführungsbeispiel 5

### Synthese von α-Tocopherol-acetat:

Man arbeitet wie in Beispiel 5 beschrieben, bei gleicher Stöchiometrie der Edukte, nur daß man statt n-Propylacetat als Lösungsmittel 15 ml Isobutylacetat einsetzt und zur Reaktionsmischung 0,83 ml Wasser hinzufügt. Nach 6 stündiger Reaktionszeit bei 50 °C arbeitet man wie gewöhnlich auf und erhält ein Produkt in Form eines hellbraunen Öls, das laut HPLC zu 20,1 wt% aus Vitamin E und zu 67,71 wt% aus Vitamin E-Acetat besteht. Das entsprechende Monoacetat wird mit einem Gehalt von 2,0 wt% detektiert. Damit ergibt sich eine Ausbeute von 90 % der Theorie.

### Ausführungsbeispiel 6

### Synthese von α-Tocopherolacetat

Man arbeitet wie in Beispiel 4 beschrieben und legt 5,2 g (= 38,17 mmol) ZnCl₂ und 8,9 g (= 45,8 mmol) TMHQ-Monoacetat in 15 ml Methylisobutylketon vor, fügt zu dieser Mischung 0,63 ml konzentrierte HCl hinzu und erhitzt auf 35-40°C. Unter Beibehaltung dieser Temperatur wird innerhalb von 2,5 h 13,8 g (= 46,5 mmol) Isophytol zudosiert und weitere 2,5 h gerührt. Nach dem Abkühlen wird mit 10 ml Wasser gewaschen. Zur organischen Phase werden 60 ml Toluol gegeben, mit 20 ml einer 2% NaOH Lösung gewaschen, nochmal hintereinander mit je 20 ml Wasser nachgewaschen, getrocknet und schließlich am Rotationsverdampfer eingeengt. Man erhält 19,1 g eines braunen, sirupartigen Öls, das laut quantitativer HPLC Bestimmung einen α-Tocopherolacetat-Gehalt von 47,7 wt% besitzt. Dies entspricht einer Ausbeute von 42,1%. Bei einem Umsatz von 85,4% entspricht das einer Selektivität von TMHQ-MA zu Tocopherolacetat von 49,3%. Freies Tocopherol wird nur im Spurenbereich detektiert.

### Ausführungsbeispiel 7

### Synthese von α-Tocopherolacetat aus TMHQ-MA:

Man arbeitet wie in Beispiel 4 beschrieben und legt 5,2 g (= 38,17 mmol) ZnCl₂ und 8,9 g (= 45,8 mmol) TMHQ-Monoacetat in 15 ml Methylisobutylketon vor, fügt zu dieser Mischung 0,63 ml konzentrierte HCl hinzu und erhitzt diese Mischung auf 50°C. Unter Beibehaltung dieser Temperatur wird innerhalb von 2,5 h 13,8 g (= 46,5 mmol) Isophytol zudosiert und weitere 2,5 h gerührt. Nach dem Abkühlen wird mit 10 ml Wasser gewaschen. Zur organischen Phase werden 60 ml Toluol gegeben, mit 20 ml einer 2% NaOH Lösung gewaschen, nochmal hintereinander mit je 20 ml Wasser nachgewaschen, getrocknet und schließlich am Rotationsverdampfer eingeengt. Man erhält 21,7 g eines braunen, sirupartigen Öls, das laut quantitativer HPLC Bestimmung einen α-Tocopherolgehalt von 13,15 wt% besitzt, der Gehalt an Tocopherolacetat beträgt 80,42 wt%. Nach Umrechnung des Vitamin E Gehalts in sein entsprechendes Vitamin E-Acetat Äquivalent ergibt dies eine Ausbeute von 20,58 g oder 95,1%.

### Ausführungsbeispiel 8

### Synthese von α-Tocopherolacetat aus TMHQ-DA:

Man arbeitet wie in Beispiel 1 beschrieben, benutzt als Protonensäure jedoch nicht HCl_{konz} sondern leitet in die Reaktionslösung während der Reaktionszeit kontinuierlich HCl_{Gas} ein und benutzt zum Auskreisen des Reaktionswassers einen Wasserabscheider. Man legt 9,2 g (= 67,5 mmol = 68,4 mol% bezogen auf TMHQ-DA) ZnCl₂ und 23,6 g (= 98,62 mmol) TMHQ-Diacetat in 100 ml Toluol vor, fügt zu dieser Mischung 0,17 g Octadecylamin hinzu und erhitzt diese Mischung auf Rückflußtemperatur. Unter Beibehaltung dieser Temperatur wird innerhalb von 2,5 h 29,6 g (= 99,8 mmol) Isophytol zudosiert und weitere 2,5 h gerührt. Während der Isophytolzudosierung und der Nachreaktionszeit wird in die Reaktionslösung HCl eingeleitet. Man kühlt ab, nimmt die Rohmischung in 400 ml Petrolether auf und wäscht in der üblichen Reihenfolge mit entsprechenden Mengen Wasser und NaHCO₃-Lösung. Die resultierende organische Phase wird am Rotationsverdampfer eingeengt. Man erhält 41,0 g eines braunen, sirupartigen Öls, das laut quantitativer HPLC Bestimmung einen α-Tocopherolgehalt von 2,9 wt% besitzt, der Gehalt an Tocopherolacetat beträgt 69,5 wt%. Nach Umrechnung des Vitamin E Gehalts in sein entsprechendes Vitamin E-Acetat Equivalent ergibt dies eine Ausbeute von 29,8 g oder 63,9%.

### Vergleichsbeispiel 1 nach EP 0 694 541

### Synthese von α-Tocopherol aus TMHQ:

Man verfährt wie im Patent beschrieben und legt 23,3 g (= 153 mmol) TMHQ und 17,5 g (= 129 mmol) ZnCl₂ und 2,2 ml HCl_{konz} in 54 ml Methylisobutylketon als Lösungsmittel vor: Zu dieser Lösung gibt man während 3 h 46,1 g (= 153 mmol) Isophytol hinzu. Die Reaktionstemperatur beträgt 25-30°C, die Reaktionsdauer beträgt 5 h. Nach üblicher Aufarbeitung erhält man das Produkt als braunes Öl mit einer Auswaage von 61,8g. Nach Gehaltsbestimmung mittels quantitativer HPLC besitzt das Öl einen α-Tocopherolgehalt von 91,4 wt%. Dies entspricht einer Ausbeute von 78,1 %.

### Ausführungsbeispiel 9-13

### Synthese von α-Tocopherolacetat:

Die folgenden Versuche wurden durchgeführt, um den Einfluß eines Cosolvens zu dem als Lösungsmittel benutzten Heptan zu untersuchen: Man arbeitet wie in Beispiel 1 beschrieben, benutzt als Protonensäure jedoch HBr_{konz}; es wird kein Kondensations-wasser bzw. Essigsäure ausgekreist. Man legt 8,6 g (= 38,2 mmol = 82,9 mol% bezogen auf TMHQ-DA) ZnBr2 und 10,9 g (= 46,1 mmol) TMHQ-Diacetat in 10 ml Heptan vor. Als Cosolvens wird je nach Versuch 1 ml des entsprechenden Alkohols hinzugefügt. Zu dieser Mischung werden 1,3 ml 47% HBr (= 11,49 mmol = 24,9 mol%) hinzugefügt. Bei 60°C wird nun während 1,5 h 105 mol% (=14,5 g = 48,9 mmol) Isophytol zudosiert. Unter Beibehaltung dieser Temperatur wird 2 h nachgerührt. Man kühlt ab und arbeitet wie gewöhnlich auf. Die folgende Tabelle gibt Ausbeuten und Gehalte an Vitamin E und Vitamin E-Acetat dieser Versuchsreihe wieder:

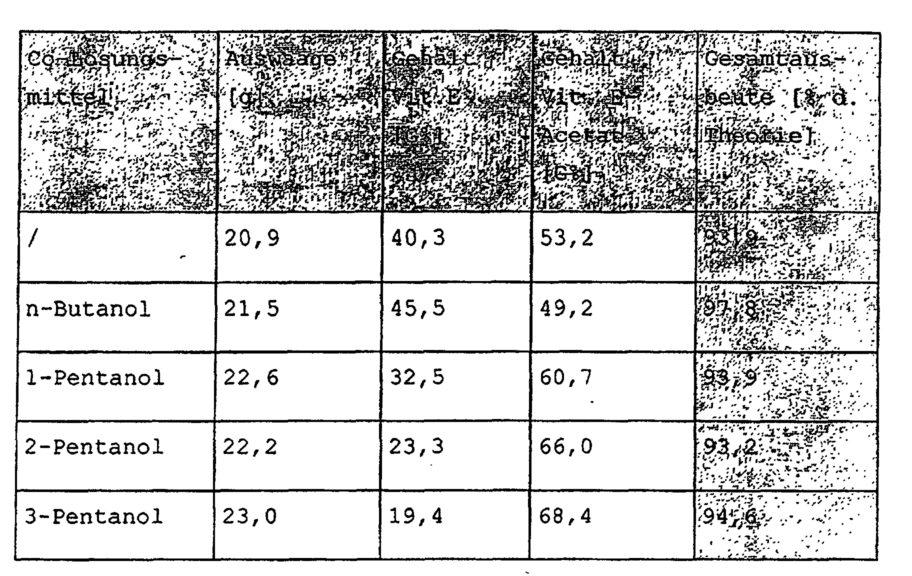

### Ausführungsbeispiel 14-21

### Synthese von α-Tocopherylacetat:

Die folgenden Versuche wurden durchgeführt, um den Einfluß der ZnCl₂-Konzentration zu untersuchen: Man arbeitet wie in Beispiel 1 beschrieben, benutzt als Protonensäure jedoch HCl_{konz}; es wird kein Kondensationswasser bzw. entstehende Essigsäure ausgekreist. Man legt die in der nachfolgenden Tabelle angeführten Mengen ZnCl₂ und 10,9 g (= 46,1 mmol) TMHQ-Diacetat in Isobutylacetat vor. Zu dieser Mischung werden 0,63 ml 37% HCl (= hinzugefügt. Bei 60°C wird nun während 4,0 h 105 mol% (=14,5 g = 48,9 mmol) Isophytol zudosiert. Unter Beibehaltung dieser Temperatur wird 2 h nachgerührt. Man kühlt ab und arbeitet wie gewöhnlich auf. Die folgende Tabelle gibt Ausbeuten und Gehalte an Vitamin E und Vitamin E-Acetat dieser Versuchsreihe wieder:

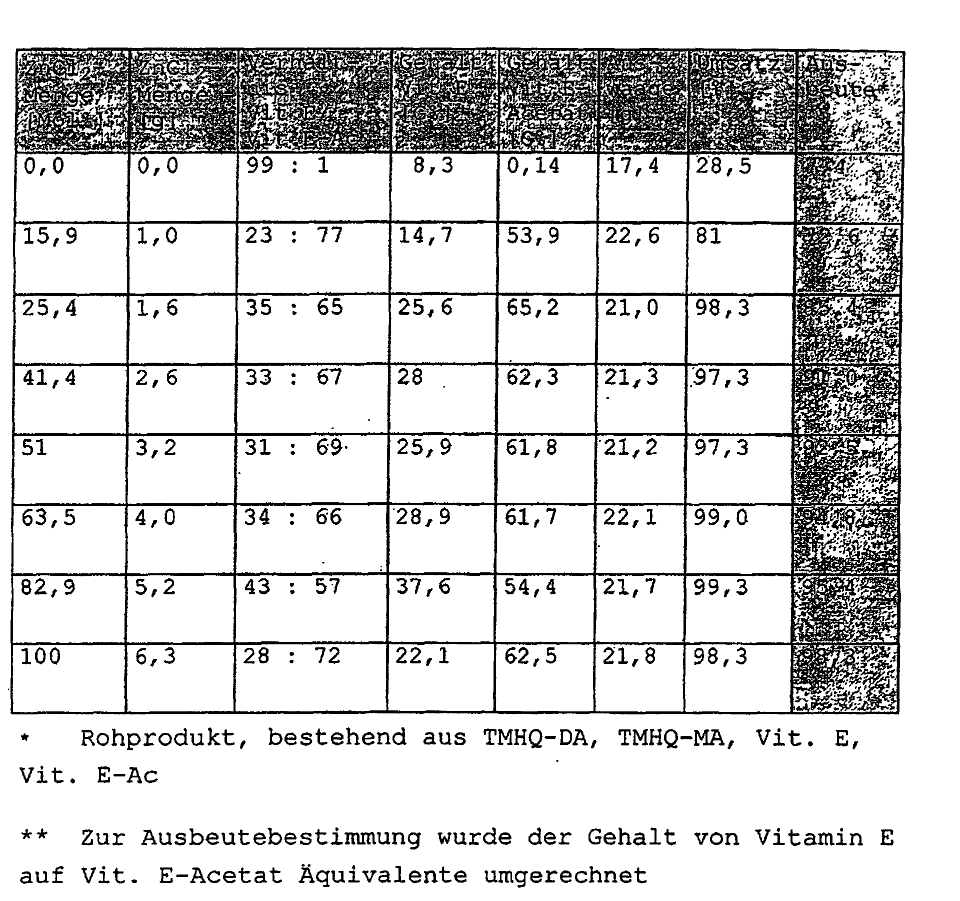

- Standardversuch:: 10,9 g TMHQ-DA (100%) = 46,1 mmol
14,5 Isophytol (BASF: GC: >98%)
= 48,9 mmol
Protonensäure: 0,63 ml HCl_{konz}.
Lewissäure: X g ZnCl_{2,} Temperatur: 60 °C
Reaktionszeit: 6 h

### Ausführungsbeispiel 22-28:

### Synthese von α-Tocopherolacetat:

Die folgenden Versuche wurden durchgeführt, um den Einfluß der ZnBr₂-Konzentration zu untersuchen: Man arbeitet wie in Beispiel 15-22 beschrieben, unter Benutzung von HBr_{konz} als Protonensäure. Es wird kein Kondensationswasser bzw. entstehende Essigsäure ausgekreist. Man legt die in der nachfolgenden Tabelle angeführten Mengen ZnBr₂ und 10,9 g (= 46,1 mmol) TMHQ-Diacetat in einem Lösungsmittelgemisch aus Heptan und Butanol vor. Zu dieser Mischung werden 1,3 ml 47% % HBr hinzugefügt. Bei 60°C wird nun während 2,0 h 105 mol% (=14,5 g = 48,9 mmol) Isophytol zudosiert. Unter Beibehaltung dieser Temperatur wird 2 h nachgerührt. Man kühlt ab und arbeitet wie gewöhnlich auf. Die folgende Tabelle gibt Ausbeuten und Gehalte an Vitamin E und Vitamin E-Acetat dieser Versuchsreihe wieder:

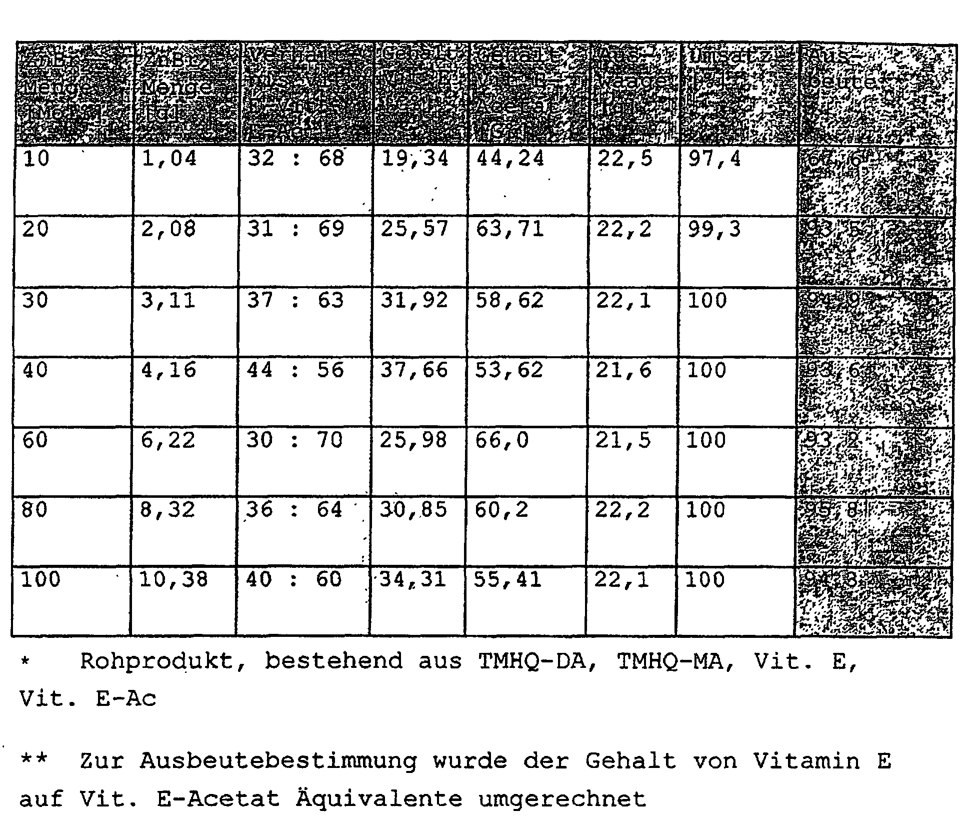

- **Standardversuch:**: 10,9 g TMHQ-DA (100%) = 46,1 mmol
14,5 Isophytol (BASF: GC: >98%) =
48,9 mmol
Protonensäure: 1,3 ml HBr_{konz}.
Lewissäure: X g ZnBr_{2,} Temperatur:
60 °C, Reaktionszeit: 4 h,
Lösungsmittel: 15 ml Heptan; 1,5 ml n-Butanol

### Ausführungsbeispiel 29-38

### Synthese von α-Tocopherolacetat

Die folgenden Versuche wurden durchgeführt, um den Einfluß der Art der Protonensäure in Gegenwart von Zinkbromid als Lewissäure zu untersuchen. Mit einbezogen in die Untersuchung wurden sowohl Mineralsäuren als auch organische Säuren. Die Versuche wurden wie folgt durchgeführt: Man legt TMHQ-DA in 15 ml Heptan und 1,5 ml Butanol als Cosolvens vor und gibt die entsprechende Menge wässriger Säure und ZnBr2 als Lewis-Säure hinzu. Das Gemisch wird auf dem Wasserbad auf 60 °C erwärmt und nach einer 15 min. Vorlaufzeit gibt man Isophytol während 3 h kontinuierlich zu. Nach einer Nachreaktionszeit von weiteren 3h wird wie üblich aufgearbeitet. Die so erhaltenen Ausbeuten und Umsätze sind in der folgenden Tabelle zusammengefasst:

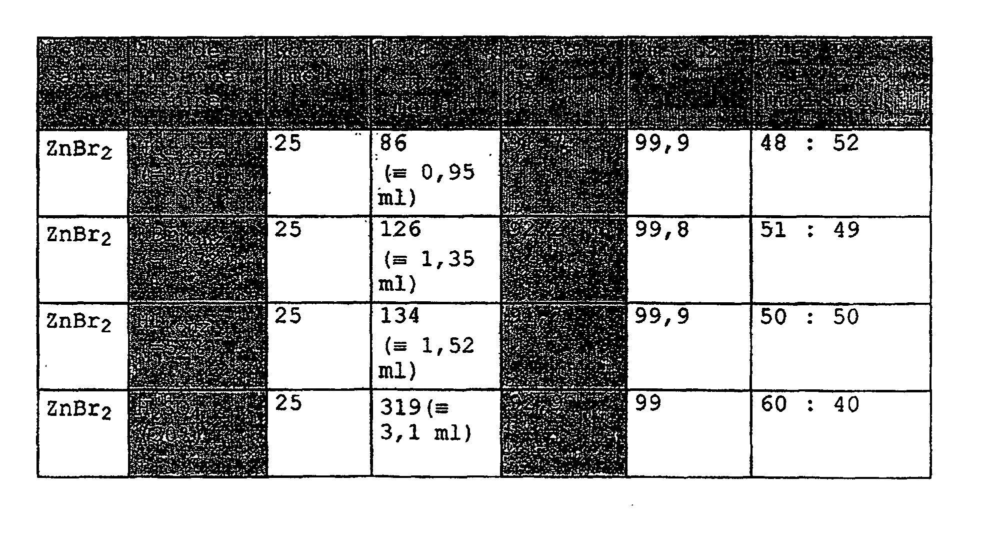

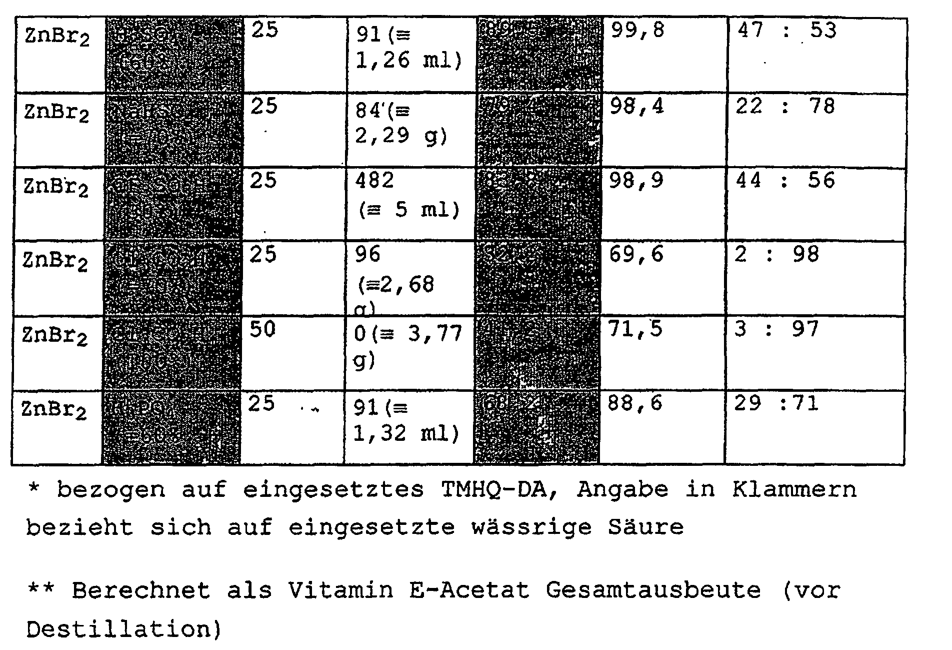

- Standardversuch:: 10,9 g TMHQ-DA (100%) = 46,1 mmol 15 ml Heptan, 1 ml Butanol reinst 8,6 ZnBr₂ (= 38,2 mmol = 82,9 mol%) 14,5 Isophytol (BASF: GC: >98%) = 48,9 mmol Reaktionstemperatur: 60 °C

### Ausführungsbeispiel 39/40:

### Synthese von α-Tocopherolacetat

Man legt TMHQ-DA, Lewissäure und die Protonensäure in entsprechenden Mengen Propylencarbonat vor. Das Gemisch wird auf dem Wasserbad auf 60 °C erwärmt und nach einer 15 min. Vorlaufzeit gibt man Isophytol während 3 h kontinuierlich zu. Das stöchiometrische Verhältnis von TMHQ-DA/Isophytol beträgt bei dieser Versuchsreihe 3:1. Variiert wird die Lösungsmittelmenge. Nach einer Nachreaktionszeit von weiteren 3h wird die Propylencarbonatphase mit Petrolether extrahiert. Beide Phasen werden analysiert. Nicht umgesetzte TMHQ-Ester in der Propylencarbonatphase können erneut eingesetzt werden. Die so erhaltenen Ausbeuten und Umsätze sind in der folgenden Tabelle zusammengefasst:

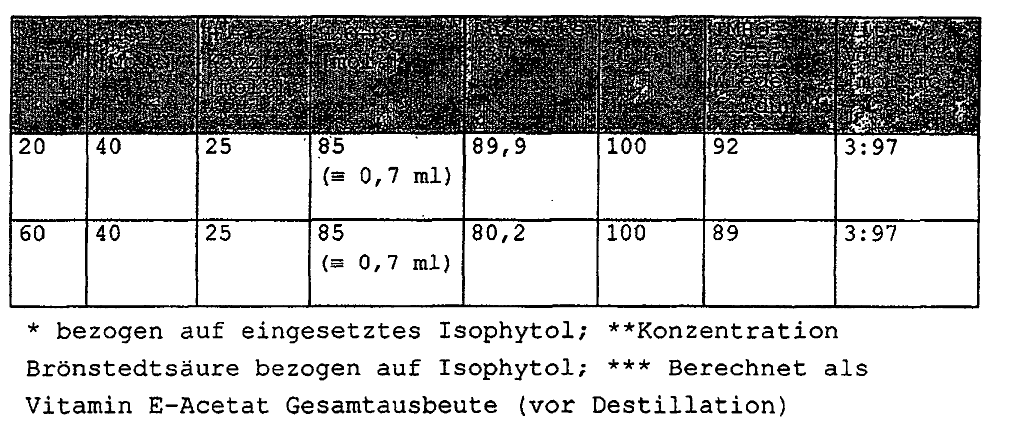

- Einwaagen:: 32,7 g = 138 mmol TMHQ-DA; 13,8 g = 46 mmol IP; 2,5 g ZnCl₂ (= 18,4 mmol = 82,9 mol%), 1,12 HCl_{Konz}

### Ausführungsbeispiel 41-45:

### Synthese von α-Tocopherolacetat

Man legt TMHQ-DA, Lewissäure und die Protonensäure in Propylencarbonat vor. Das Gemisch wird auf dem Wasserbad erwärmt und nach einer 15 min. Vorlaufzeit gibt man Isophytol während 3 h kontinuierlich zu. Bei dieser Versuchsreihe wird mit einem 5 mol% Überschuß an Isophytol bezogen auf TMHQ-DA gearbeitet.Nach einer Nachreaktionszeit von weiteren 3h wird die Propylencarbonatphase mit Petrolether extrahiert. Die Ausbeuteangaben lassen Vit. E bzw. Vit.E-Acetat in der Propylencarbonatphase unberücksichtigt. Die so erhaltenen Ausbeuten und Umsätze sind in der folgenden Tabelle zusammengefasst:

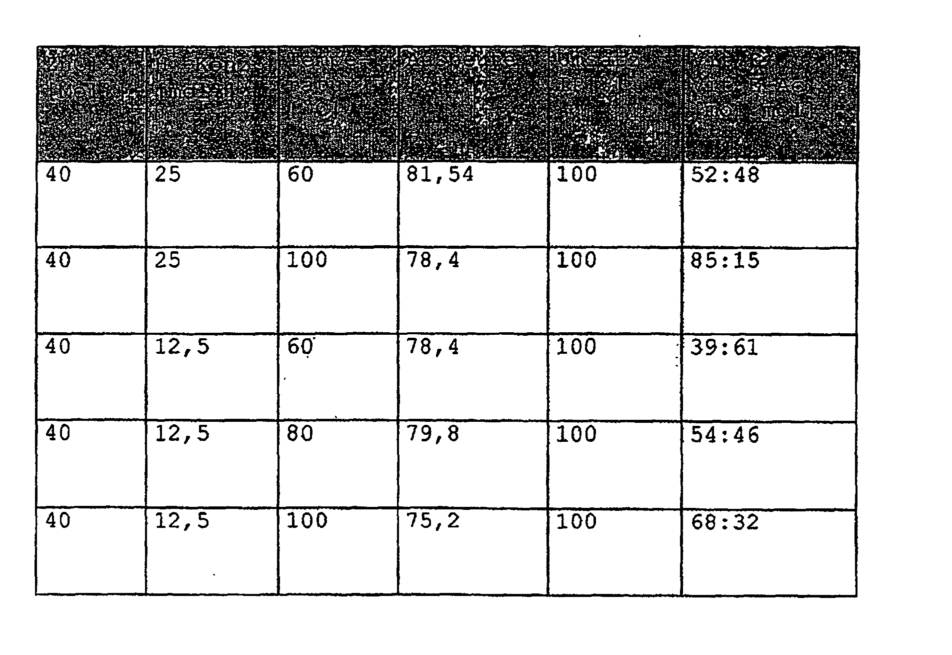

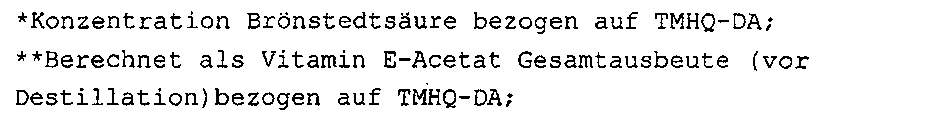

- Einwaagen:: 10,9 g = 46 mmol TMHQ-DA; 14,75 g = 49 mmol IP; 2,5 g ZnCl₂ (= 18,4 mmol), 0,62g H₂O (= 75 Mol% H₂O);H⁺-Kat. = H₃BO₃/C₂O₄H₂ (Molar: 1:2) 12,5 - 25 Mol%

### Ausführungsbeispiel 46-51:

### Synthese von α-Tocopherolacetat

Die folgenden Versuche wurden durchgeführt, um den Einfluß der H₂O-Konzentration und der Brönstedtsäurekonzentration in Gegenwart von Zinkbromid als Lewissäure zu dokumentieren. Die Versuche wurden wie folgt durchgeführt: Man legt TMHQ-DA in 15 ml Heptan und 1,5 ml Butanol als Cosolvens vor und gibt die entsprechende Menge wässriger Säure und ZnBr₂ als Lewissäure hinzu. Das Gemisch wird auf dem Wasserbad auf 60 °C erwärmt und nach einer 15 min. Vorlaufzeit gibt man Isophytol während 3 h kontinuierlich zu. Nach einer Nachreaktionszeit von weiteren 3h wird wie üblich aufgearbeitet. Die so erhaltenen Ausbeuten und Umsätze sind in der folgenden Tabelle zusammengefasst:

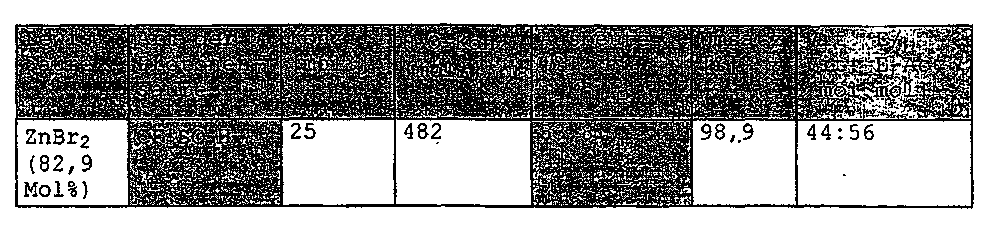

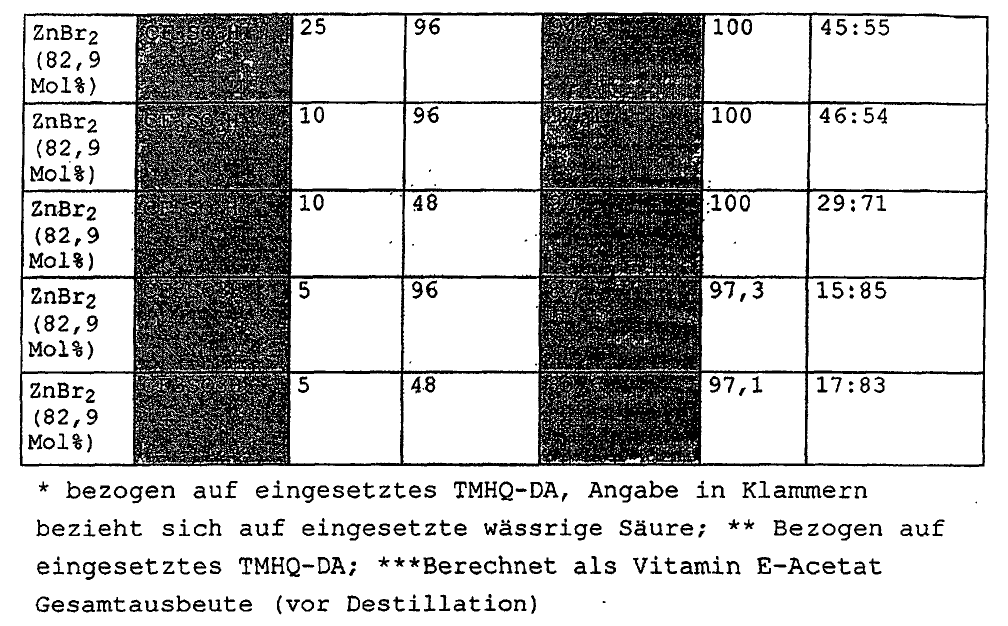

- Standardversuch:: 10,9g TMHQ-DA (100%) = 46,1 mmol; 15 ml Heptan, 1 ml Butanol reinst; 8,6 ZnBr₂ (= 38,2 mmol = 82,9 mol%); 14,5 g Isophytol (BASF:GC:>98%) = 48,9mmol

### Vergleichsbeispiel 2:

### Darstellung von DL-α-Tocopherolacetat nach JP-OS Sho 51-80859 bei 125°C:

In einem 200 ml Dreihalskolben mit Rührer, Rückflußkühler, Thermometer und Tropftrichter legt man 15,5 g(= 65,6 mmol) TMHQ-DA und 12,0 g (= 88mmol) Zinkchlorid in 20 ml Butylacetat vor. Man hält die so erhaltene Mischung im Stickstoffstrom unter Rückfluß, wobei die Rückflußtemperatur 125°C beträgt. Zu dieser siedenden Mischung tropft man innerhalb 50 min. 37,7 g (= 127,3 mmol) Isophytol zu, die in weiteren 10 ml Butylacetat gelöst sind. Danach wird die Reaktion weitere 4 h unter Rückfluß gehalten. Nach Ablauf der Reaktion wird die Reaktionsmischung mit Wasser extrahiert, wobei das Zinkchlorid in die wässrige Phase übergeht und eine Tocopherol-/Tocopheryl-acetat- Mischung in der organischen Phase verbleibt. Nach der gaschromatographischen Analyse ist der Umsatz von TMHQ-DA vollständig, die Rohausbeute an Tocopherol beträgt 22,5%, die Ausbeute an Tocopherolacetat beträgt 45,8%, insgesamt ist die Ausbeute an Tocopherol/Tocopherolacetat also 68% bezogen auf TMHQ-DA.

### Vergleichsbeispiel 3:

### Darstellung von DL-α-Tocopherolacetat nach JP-OS Sho 51-80859 bei 60°C (Katalysator = ZnCl₂ ohne Protonensäure):

Man verfährt wie im Vergleichsbeispiel 1 beschrieben mit dem Unterschied, daß man die Reaktionstemperatur auf 60°C herabsetzt. Nach üblicher Aufarbeitung und gaschromatographischer Analyse der organischen Bestandteile der Mischung erhält man das Ausgangsmaterial TMHQ-DA zu 100% zurück. Die Reaktion findet also unter diesen Bedingungen nicht statt.

## Patentansprüche

1. Verfahren zur Herstellung von α-Tocopherolestern, seinen Derivaten oder -homologen gemäß der allgemeinen Formel 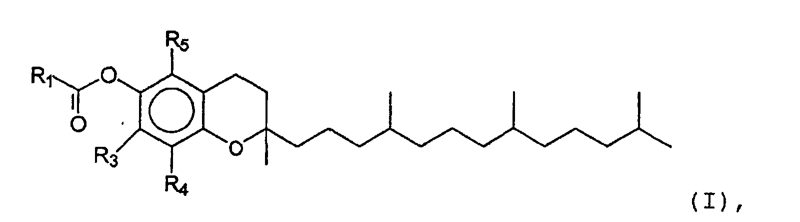 **dadurch gekennzeichnet,**
**daß** man den Mono- oder Diester eines Hydrochinons gemäß der allgemeinen Formel 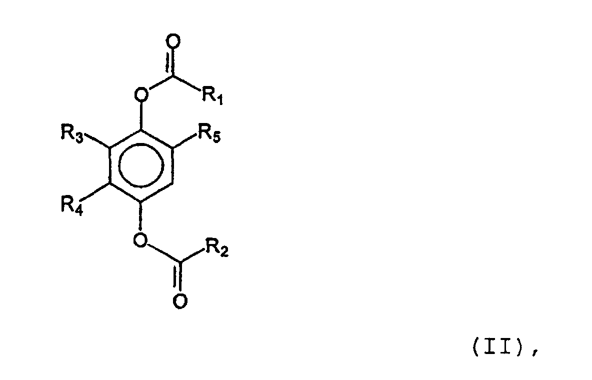 in der bedeuten:
R₁, R₂: Alkyl mit C₁-C₂₀, verzweigt oder unverzweigt,gesättigt oder ungesättigt
R₃, R₄, R₅: H, Alkyl mit C₁-C₃, gleich oder verschieden,
mit einem Allylalkoholderivat der allgemeinen Formel 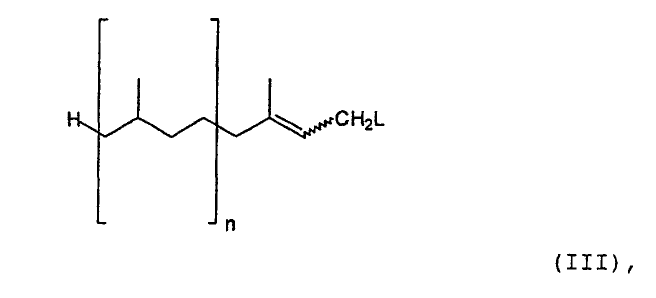 in der n eine Zahl von 0 bis 5 und L einer Hydroxyl-, Halogen-, Acetoxy-, Methansulfonyloxy-, Ethansulfonyloxy-, Benzolsulfonyloxy- oder Toluol-sulfonylgruppe entspricht,
oder mit einem Allylalkohol der allgemeinen Formel 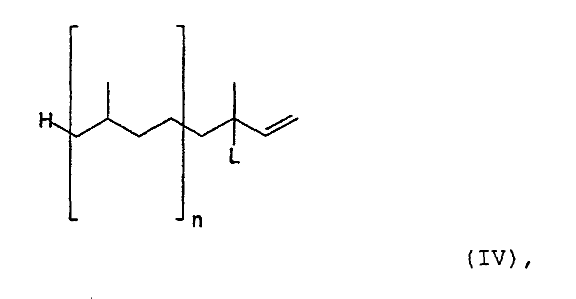 in der n dieselben Zahlen repräsentiert wie oben und L einer Hydroxyl-, Halogen- oder Acetoxygruppe entspricht,
in Gegenwart von Zinkhalogeniden und Protonen abspaltenden Säuren bei einer Temperatur von 25 bis 100 °C zur Reaktion bringt.

2. Verfahren gemäß Anspruch 1,
**dadurch gekennzeichnet,**
**daß** man die Umsetzung in Gegenwart von 10⁻² bis 100 Mol% Zinkhalogenid, bezogen auf den eingesetzten TMHQ-Diester, insbesondere das Diacetat, durchführt.

3. Verfahren gemäß den Ansprüchen 1 und 2,
**dadurch gekennzeichnet,**
**daß** man als Protonensäure Salzsäure oder Bromwasserstoffsäure oder Schwefelsäure oder Trifluormethansulfonsäure oder eine Mischung aus Borsäure und Oxalsäure einsetzt.

4. Verfahren gemäß den Ansprüche 1 bis 3,
**dadurch gekennzeichnet,**
**daß** man die Protonensäure in einer Konzentration von 1 bis 100 Mol%, bezogen auf TMHQ-Diacetat, einsetzt.

5. Verfahren gemäß den Ansprüchen 1 bis 4,
**dadurch gekennzeichnet,**
**daß** man 0,1 bis 100 ml Lösungsmittel/g TMHQ-Diacetat, insbesondere 1 bis 10 ml/g, einsetzt.

6. Verfahren gemäß den Ansprüchen 1 bis 5,
**dadurch gekennzeichnet,**
**daß** man als Lösungsmittel cyclische oder aliphatische Carbonatester von Alkoholen mit einer Kohlenstoffkette von C-1 bis C-4, oder Carbonsäureester oder nicht polare Lösungsmittel wie aliphatische Kohlenwasserstoffe oder aromatische Kohlenwasserstoffe einsetzt.

7. Verfahren gemäß den Ansprüchen 1 bis 6,
**dadurch gekennzeichnet,**
**daß** man das TMHQ-Diacetat und das Isophytol in einem molaren Verhältnis von 1 : 1 bis 10 : 1 umsetzt.

8. Verfahren gemäß den Ansprüchen 1 bis 7,
**dadurch gekennzeichnet,**
**daß** man die Umsetzung kontinuierlich durchführt.

9. Verfahren gemäß den Ansprüchen 1 bis 8,
**dadurch gekennzeichnet,**
**daß** das entstehende Reaktionswasser während der Reaktion zumindest zum Teil in der Reaktionsmischung verbleibt.

10. Verfahren gemäß den Ansprüche 1 bis 9,
**dadurch gekennzeichnet,**
**daß** man den Ester gemäß Formel (II), das Zinkhalogenid und die Protonen abspaltenden Säure in einem Lösungsmittel vorlegt und dieser Mischung die Verbindung gemäß Formel (III) oder (IV) zusetzt.

## Claims

1. Process for the production of α-tocopherol esters, the derivatives or homologues thereof of the general formula 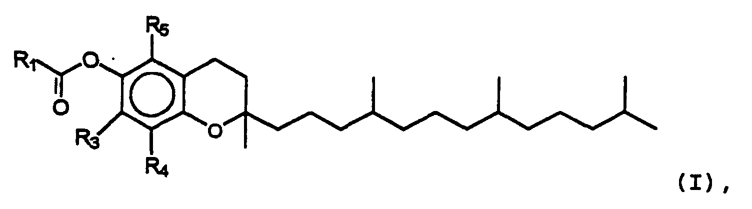 **characterised in that**
the mono- or diesters of a hydroquinone of the general formula 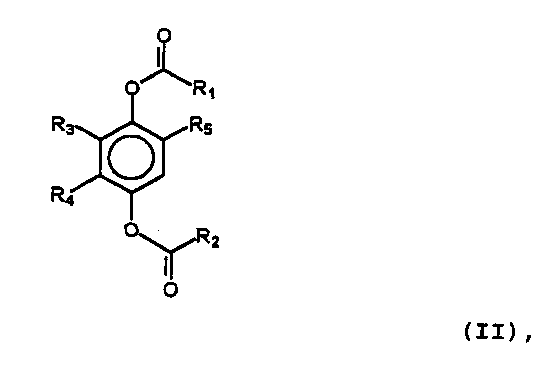 in which
R₁, R₂ mean C₁-C₂₀ alkyl, branched or unbranched, saturated or unsaturated
R₃, R₄, R₅ mean H, C₁-C₃ alkyl, identical or different
are reacted with an allyl alcohol derivative of the general formula 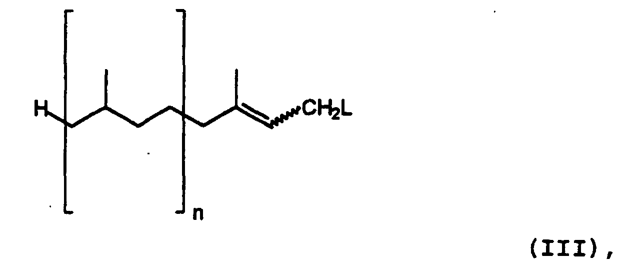 in which n represents a number from 0 to 5 and L represents a hydroxyl, halogen, acetoxy, methanesulfonyloxy, ethanesulfonyloxy, benzenesulfonyloxy or toluenesulfonyl group,
or with an allyl alcohol of the general formula 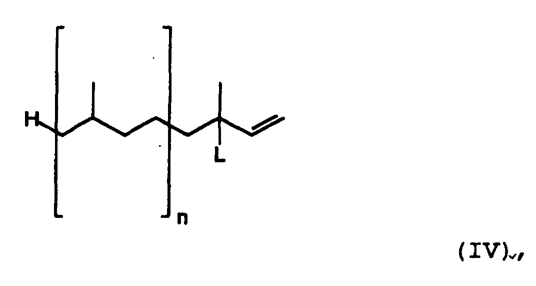 in which n represents the same numbers as above and L represents a hydroxyl, halogen or acetoxy group,
in the presence of zinc halides and proton-liberating acids at a temperature of 25 to 100 °C.

2. Process according to claim 1,
**characterised in that**
the reaction is performed in the presence of 10⁻² to 100 mol% of zinc halide, relative to the introduced TMHQ diester, in particular the diacetate.

3. Process according to claims 1 and 2
**characterised in that**
the protonic acid used is hydrochloric acid or hydrobromic acid or sulfuric acid or trifluoromethanesulfonic acid or a mixture of boric acid and oxalic acid.

4. Process according to claims 1 to 3,
**characterised in that**
the protonic acid is used in a concentration of 1 to 100 mol%, relative to TMHQ diacetate.

5. Process according to claims 1 to 4,
**characterised in that**
0.1 to 100 ml of solvent/g of TMHQ diacetate, in particular 1 to 10 ml/g, is used.

6. Process according to claims 1 to 5,
**characterised in that**
the solvents used are cyclic or aliphatic carbonate esters of alcohols having a C-1 to C-4 carbon chain, or carboxylic acid esters or non-polar solvents such as aliphatic hydrocarbons or aromatic hydrocarbons.

7. Process according to claims 1 to 6,
**characterised in that**
the TMHQ diacetate and isophytol are reacted in a molar ratio of 1:1 to 10:1.

8. Process according to claims 1 to 7,
**characterised in that**
the reaction is performed continuously.

9. Process according to claims 1 to 8,
**characterised in that**
the water of reaction formed during the reaction at least in part remains in the reaction mixture.

10. Process according to claims 1 to 9,
**characterised in that**
the esters of the formula (II), the zinc halide and the proton-liberating acid are initially introduced in a solvent and the compound of the formula (III) or (IV) is added to this mixture.

## Revendications

1. Procédé de préparation d'esters d'α-tocophénol, de leurs dérivés ou homologues conformément à la formule générale (I) 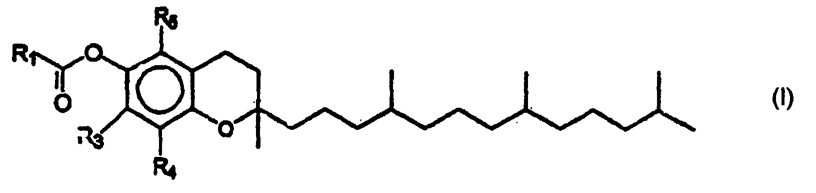 **caractérisé en ce qu'**
on met en réaction le mono- ou le diester d'une hydroquinone de formule générale (II) : 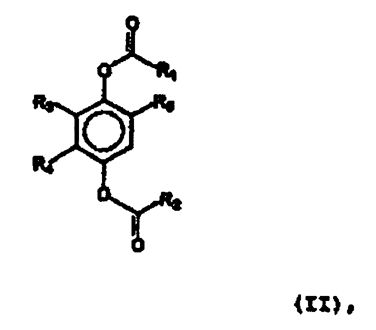 dans laquelle
R₁ et R₂ signifient : un alkyle en C₁ à C₂₀, ramifié ou non ramifié, saturé ou non saturé,
R₃, R₄, R₅ signifient H, alkyle en C₁-C₃, identiques ou différents,
avec un dérivé d'alcool allylique de formule générale (III) : 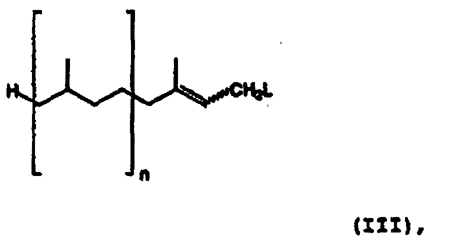 dans laquelle n correspond à un nombre allant de 0 à 5 et L correspond à un groupe hydroxyle, halogène, acétoxy, methane-sulfonyloxy, éthane-sulfonyloxy, benzène-sulfonyloxy, ou toluène-sulfonyloxy,
ou avec un alcool allylique de formule générale : 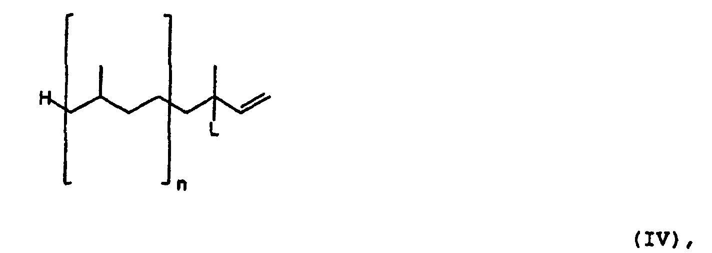 dans laquelle n représente les mêmes chiffres que ci-dessus et L correspond à un groupe hydroxyle, halogène ou acétoxy,
en présence d'halogénures de zinc et d'acide qui détache les protons à une température allant de 25 à 100°C.

2. Procédé selon la revendication 1,
**caractérisé en ce qu'**
on effectue la réaction en présence de 10⁻² à 100 % molaire d'halogène de zinc rapporté au diester de TMHQ mis en oeuvre, en particulier le diacétate.

3. Procédé conformément aux revendications 1 et 2,
**caractérisé en ce qu'**
on met en oeuvre comme acide protoné de l'acide chlorhydrique ou de l'acide bromhydrique ou de l'acide sulfurique ou de l'acide trifluorométhane sulfonique ou bien un mélange à base d'acide borique et d'acide oxalique.

4. Procédé conformément à l'une quelconque des revendications 1 à 3,
**caractérisé en ce qu'**
on met en oeuvre l'acide protoné à une concentration de 1 à 100 % molaire rapporté au diacétate de TMHQ.

5. Procédé conformément à l'une quelconque des revendications 1 à 4,
**caractérisé en ce qu'**
on met en oeuvre de 0,1 à 100 ml de solvant/g de diacétate de TMHQ, en particulier de 1 à 10 ml/g.

6. Procédé conformément à l'une quelconque des revendications 1 à 5,
**caractérisé en ce qu'**
on met en oeuvre comme solvant des esters de carbonate cyclique ou aliphatique et d'alcools ayant une chaîne carbonée allant de C₁ à C₄ ou des esters et acide carboxylique ou des solvants non polaires comme des hydrocarbures aliphatiques ou des hydrocarbures aromatiques.

7. Procédé conformément à l'une quelconque des revendications 1 à 6,
**caractérisé en ce qu'**
on fait réagir le diacétate de TMHQ et l'isophytol dans un rapport molaire de 1 : 1 à 10 : 1.

8. Procédé conformément à l'une quelconque des revendications 1 à 7,
**caractérisé en ce qu'**
on effectue la réaction en continu.

9. Procédé conformément à l'une quelconque des revendications 1 à 8,
**caractérisé en ce que**
l'eau de réaction qui se forme pendant la réaction demeure au moins en partie dans le mélange réactionnel.

10. Procédé conformément à l'une quelconque des revendications 1 à 9,
**caractérisé en ce qu'**
on introduit l'ester de formule (II), l'halogénure de zinc et l'acide qui détache les protons dans un solvant et on ajoute à ce mélange le composé conformément à la formule (III) ou (IV).
